Europäisches Patentamt

**European Patent Office**

**Office européen des brevets**

(19)

(11) Publication number: **0 291 633 B1**

## EUROPEAN PATENT SPECIFICATION

(12)

(45) Date of publication of patent specification: **04.11.92**   (51) Int. Cl.⁵: **A61K  31/70**, C07H 19/073

(21) Application number: **88101790.9**

(22) Date of filing: **14.03.86**

(60) Publication number of the earlier application in accordance with Art.76 EPC: **0 196 185**

(54) **Use of 3'-azido-3'-deoxythymidine in the treatment or prophylaxis of human retroviral infections.**

(30) Priority: **16.03.85 GB 8506869**
         **09.05.85 GB 8511774**
         **27.09.85 GB 8523881**
         **12.02.86 GB 8603450**
         **17.09.85 US 776899**

(43) Date of publication of application:
     **23.11.88 Bulletin  88/47**

(45) Publication of the grant of the patent:
     **04.11.92 Bulletin  92/45**

(84) Designated Contracting States:
     **AT BE CH DE FR GB IT LI LU NL SE**

(56) References cited:

     **EXPERIMENTAL CELL RESEARCH, vol. 116, no. 1, 1978, Academic Press Inc. (SE); W.OSTERTAG et al., pp. 31-37***

     **EXPERIMENTAL CELL RESEARCH, vol. 116, no. 1, 1978, Academic Press Inc. (SE); C.J.KRIEG et al., pp. 21-29***

     **PROCEEDINGS OF THE NATL. ACADEMY OF SCIENCE USA, vol. 71, no. 12, December 1974; W.OSTERTAG et al., pp. 4980-4985***
(73) Proprietor: **THE WELLCOME FOUNDATION LIMITED**
     **Unicorn House 160 Euston Road**
     **London NW1 2BP(GB)**

(72) Inventor: **Rideout, Janes Litster**
     **3101 Morningside Drive**
     **Raleigh, NC(US)**
     Inventor: **Barry, David Walter**
     **1810S Lakeshore Drive**
     **Chapel Hill, NC(US)**
     Inventor: **Lehrman, Sandra Nusinoff**
     **2720 Old Sugar Road**
     **Durham, NC(US)**
     Inventor: **St Clair, Martha Heider**
     **312 Seven Oaks Road**
     **Durham, NC(US)**
     Inventor: **Furman, Phillip Allen**
     **901 Bluestone Road**
     **Durham, NC(US)**

(74) Representative: **Garrett, Michael et al**
     **The Wellcome Research Laboratories Group**
     **Patents and Agreements Langley Court**
     **Beckenham Kent BR3 3BS(GB)**

PROCEEDINGS OF THE NATL. ACADEMY OF SCIENCE USA, vol. 82, October 1985; H.MITSUYA et al., pp. 7096-7100*

DIALOG INFORMATION SERVICES, file 267; DeHaen Drug Data, 1980-1987, & 25TH INTER-SCIENCE CONFERENCE ANTIMICROBIAL AGENTS CHEMOTHERAPY, Minneapolis, MN, (US); 29 September-02 October 1985; M.H.St CLAIR et al., nos. 437-440, ref.0180178* W.D.HARDY Jr. et al., nos. 437-440, ref. 0180181* H.MITSUYA et al., no. 440, ref. 0180180* P.A.FURMAN et al., no. 440, ref. 0180179*

JOURNAL OF THE AM. MED. ASSOC., vol. 254, no. 18, 08 November 1985; D.E.RIESENBERG, pp. 2521, 2527, 2529*

DIALOG INFORMATION SERVICES, file 5; Biosis Previews 69-87; June; W.D.HARDY Jr. et al., ref. 0016194545*

CHEM. & ENG. NEWS, vol. 64, no. 4, 27 January 1986; R.K.ROBINS, pp. 28-40*

FDA DRUG BULLETIN, vol. 15, no. 3, October 1985; NN., pp. 27-32*

PROCEEDINGS OF AM. ASSOC. CANCER RES., vol. 27, 77th Meeting, March 1986; R.M.RUPRECHT et al., p. 422, no. 1675*

PROCEEDINGS OF AM. SOC. CLIN. ONCOL., vol. 5, 22nd Meeting, March 1986; R.W.KLECKER et al., p. 1, no. 4*

CANCER RESEARCH, vol. 32, no. 7, July 1972; A.W.SCHRECKER et al., pp. 1547-1558*

NATURE, vol. 323, 02 October 1986; R.M.RUPRECHT et al., pp. 467-469*

THE LANCET, vol. 1, no 8539, 25 April 1987; NN., pp. 957-958*

THE LANCET, 26 August 1989; pp. 483-484*

THE LANCET, 20 February 1988; pp. 373-376*

THE LANCET, 07 April 1990; pp. 821-822*

AM. J. MED., vol. 85, 1988; pp. 176-181*

**Description**

The present invention relates to 3'-azido-3'-deoxythymidine for use in the treatment or prophylaxis of human retroviral infections, and use of 3'-azido-3'-deoxythymidine in the manufacture of a medicament for the treatment or prophylaxis of human retrovirus infection.

Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome is incorporated into the host cell genome, allowing it to take full advantage of the host cell's transcription/translation machinery for the purposes of replication. Once incorporated, the viral DNA is virtually indistinguishable from the host's DNA and, in this state, the virus may persist for as long as the cell lives. As it is virtually invulnerable to attack in this form, any treatment must be directed at another state of the life cycle and will, of necessity, have to be continued until all virus-carrying cells have died.

A species of retrovirus has now been reproducibly isolated from patients with AIDS (Acquired Immune Deficiency Syndrome). While it has been extensively characterised, there is, as yet, no agreed name for the virus, and it is currently known either as human T-cell lymphotropic virus III (HTLV III), AIDS-associated retrovirus (ARV), or lymphadenopathy associated virus (LAV). This virus (referred to herein as HTLV III) been shown preferentially to infect and destroy T-cells bearing the OKT$^4$ surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunistic infection, such as pneumonia or virally induced cancers, and not as a direct result of HTLV III infection.

Recently, HTLV III has also been recovered from other tissue types, including B- cells expressing the T$^4$ marker, macrophages and non-blood associated tissue in the central nervous system (CNS). This latter infection has been discovered in patients expressing classical AIDS symptoms and is associated with progressive demyelination, leading to wasting and such symptoms as encephalopathy, progressive dysarthria, ataxia and disorientation.

There are at least four clinical manifestations of HTLV III infection. In the initial 'carrier' state, the only indication of infection is the presence of anti-HTLV III antibodies in the blood-stream. It is believed that such 'carriers' are capable of passing on the infection, e.g. by blood transfusion, sexual intercourse or used syringe needles. The carrier state may often never progress to the second stage characterised by persistent generalised lymphadenopathy (PGL). It is currently estimated that about 20% of PGL patients progress to a more advanced condition known as 'AIDS related complex' (ARC). Physical symptoms associated with ARC may include general malaise, increased temperature and chronic infections. This condition usually progresses to the final, fatal AIDS condition, when the patient completely loses the ability to fight infection.

The existence of these human retroviruses and others has only recently been recognised and, as the diseases with which they are linked are of a life-threatening nature, there exists an urgent need to develop ways to combat these viruses.

Various drugs have now been proposed as 'cures' for AIDS. These include antimoniotungstate, suramin, ribavirin and isoprinosine, which are either somewhat toxic or have shown no marked anti-retroviral activity. As the HTLV III genome is incorporated into the host cell DNA after infection and is virtually invulnerable to attack in this state, it will persist as long as the host cell survives, causing new infection in the meantime. Thus, any treatment of AIDS would have to be for an extended period, possibly life, requiring substances with an acceptable toxicity.

Reports have described the testing of compounds against various retroviruses, for example, Friend Leukaemia Virus (FLV), a murine retrovirus. For instance Krieg et al. (Exp. Cell Res., 116 (1978) 21-29) found that 3'-azido-3'-deoxythymidine affected the release of C-type particles and increased the formation of A-type particles of a FLV complex in in vitro experiments, and Ostertag et al. (Proc. Nat. Acad. Sci. (1974) 71, 4980-85) stated that, on the basis of antiviral activity related to the above FLV complex and a lack of cellular toxicity, 3'-azido-3'-deoxythymidine "might favourably replace bromodeoxyuridine for medical treatment of diseases caused by DNA viruses". However, De Clerq et al. (Biochem. Pharm. (1980) 29, 1849- 1851) established, six years later, that 3'-azido3'-deoxythymidine had no appreciable activity against any viruses used in their tests, including vaccinia, HSVI and varicella zoster virus (VZV).

We have now discovered that 3'-azido-3'-deoxythymidine has a surprisingly potent activity against human retroviruses, with a particularly high activity against HTLV III as demonstrated by the experimental data referred to below. Such activity renders the compound useful in the therapy of human retroviral

3

infections.

Thus, in the first aspect of the present invention, there is provided 3'-azido-3'-deoxythymidine for use in the treatment or prophylaxis of a human retrovirus infection.

Activity of 3'-azido-3'-deoxythymidine against human retroviruses has been established in various in vitro assay systems. For example, infection of the H9 human lymphoblastoid cell-line by HTLV III is effectively prevented by concentrations of 3'-azido-3'-deoxythymidine as low as 0.013 mcg/ml up to 20 hours after infection. HTLV III infection of U937 human lymphoblastoid cells, PHA- stimulated white blood cells and cultured peripheral blood lymphocytes is also prevented at similarly low concentrations In addition, 10-day challenge experiments using up to 5000 HTLV III virions per cell and cloned T4, tetanus-specific, T-helper lymphocytes, showed no decrease in cells treated with 3'- azido-3'-deoxythymidine, while untreated cells had decreased 5-fold. Cytopathic effects were also completely blocked in the same cell-line transformed by HTLV-I and super-infected with HTLV III.

Other studies using purified HTLV III reverse transcriptase have shown that the activity of this enzyme is blocked by the triphosphate of 3'-azido-3'-deoxythymidine by a competitive inhibition mechanism.

Phase I clinical trials have also shown that 3'-azido-3'-deoxythymidine is capable of crossing the blood/brain barrier in clinically effective quantities. This property is both unusual and valuable for the treatment and prophylaxis of CNS infections caused by human retroviruses.

The ability of 3'-azido-3'-deoxythymidine to modify the course of retrovirus- induced malignancy has been demonstrated in a mouse model, whereby administration of 3'-azido-3'-deoxythymidine prevented splenomegaly caused by intravenously administered Rauscher Murine Leukaemia Virus, the murine equivalent of HTLV-I. In further experiments, 3'-azido-3'-deoxythymidine has been shown to inhibit the in vitro replication of HTLV-I at concentrations as low as 0.8 mcg/ml.

In a further aspect of the present invention, there is provided the use of 3'-azido-3'-deoxythymidine in the manufacture of a medicament for the treatment or prophylaxis of a human retrovirus infection.

Examples of human retrovirus infections which may be treated or prevented in accordance with the present invention include T-cell lymphotropic retroviruses (HTLV), especially HTLV-I, HTLV-II and HTLV-III. Clinical conditions that may be treated or prevented in accordance with the invention include AIDS, AIDS-related complex, the HTLV III carrier state and HTLV-I positive leukaemia and lymphoma. Suitable patients for treatment also include those having antibodies to HTLV III, HTLV III CNS infections, PGL and ARC.

Experiments have shown that 3'-azido-3'-deoxythymidine is converted, in vivo, by the action of cellular enzymes into the 5'-monophosphate. The monophosphate is then further phosphorylated by other enzymes to form the triphosphate via the diphosphate, and other studies have demonstrated that it is the triphosphate form of 3'-azido-3'-deoxythymidine which is believed to be the effective chain terminator in the reverse transcription of HTLV III, as evidenced by its effect on avian myeloblastosis virus and Moloney murine leukaemia virus. This form also inhibits HTLV III reverse transcriptase in vitro whilst having a negligible effect on human DNA polymerase activity.

3'-Azido-3'-deoxythymidine (hereafter referred to as the active ingredient), may be administered to humans for prophylaxis or treatment of retroviral infections by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient and the nature of the infection.

In general a suitable dose will be in the range of 3.0 to 120 mg per kilogram body weight of the patient per day, preferably in the range of 6 to 90 mg per kilogram body weight per day and most preferably in the range 15 to 60 mg per kilogram body weight per day. The desired dose is preferably presented as two, three, four, five, six or or more sub-doses administered at appropriate intervals throughout the day. These sub-doses may be administered in unit dosage forms, for example, containing 5 to 1500 mg, preferably 10 to 1000 mg, and most preferably 20 to 700 mg of active ingredient per unit dosage form.

Experiments with 3'-azido-3'-deoxythymidine suggest that a dose should be administered to achieve peak plasma concentrations of the active compound of from about 1 to about 75 $\mu$M, preferably about 2 to 50 $\mu$M, most preferably about 3 to about 30 $\mu$M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone, it is preferable to present it as a pharmaceutical formulation. The formulations comprise 3'-azido-3'-deoxythymidine, together with one or more acceptable carriers therefor and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bring into association the active ingredient with liquid carriers or finely divided solid carriers or both, and then if necessary shaping the product.

Formulations suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste. Oral formulations may further include other agents conventional in the art, such as sweeteners, flavouring agents and thickeners. The above pharmaceutical formulations also include those providing sustained release of the active ingredient after oral administration.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non- aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

3'-Azido-3'-deoxythymidine may also be used in therapy in conjunction with other medicaments such as 9-[[2-hydroxy-1-(hydroxymethyl)ethoxy]methyl] guanine, 9-(2-hydroxyethoxymethyl)guanine (acyclovir), 2-amino-9-(2-hydroxyethoxymethyl)purine, interferon, e.g., $\alpha$-interferon, interleukin II, and phosphonoformate (Foscarnet) or in conjunction with other immune modulating therapy including bone marrow or lymphocyte transplants or medications such as levamisol or thymosin which serve to increase lymphocyte numbers and/or function as is appropriate.

It should be understood that in addition to the ingredients particularly mentioned above the formulations of this invention may include other agents conventional in the art of formulation.

3'-azido-3'-deoxythymidine may be prepared in conventional manner, for example as described in the following references, or by methods analogous thereto:J.R. Horwitz et al., J. Org. Chem. 29, (July 1964) 2076-78; M. Imazawa et al., J. Org. Chem, 43 (15) (1978) 3044-3048; K.A. Watanabe et al., J. Org. Chem., 45, 3274 (1980); and R.P. Glinski et al., J. Chem. Soc. Chem. Commun., 915 (1970), as well as the process described in Reference Example 8 hereinafter.

The following Examples are intended for illustration. The term 'active ingredient' as used in the Examples means the compound 3'-azido-3'-deoxythymidine.

5

Reference Example 1: Tablet Formulations

The following formulations A to C were prepared by wet granulation of the ingredients with a solution of povidone, followed by addition of magnesium stearate and compression.

Formulation A

| | | mg/tablet | mg/tablet |
|---|---|---|---|
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
| | | 500 | 300 |

Formulation C.

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, were prepared by direct compression of the admixed ingredients. The lactose used in formulation E was of the direct compression type (Dairy Crest - "Zeparox").

6

Formulation D

|  | mg/tablet |
|---|---|
| Active Ingredient | 250 |
| Pregelatinised Starch NF15 | <u>150</u> |
|  | 400 |

Formulation E

|  | mg/tablet |
|---|---|
| Active Ingredient | 250 |
| Lactose | 150 |
| Avicel | <u>100</u> |
|  | 500 |

Formulation F (Controlled Release Formulation)

The formulation was prepared by wet granulation of the ingredients (below) with a solution of povidone followed by the addition of magnesium stearate and compression.

|  |  | mg/tablet |
|---|---|---|
| (a) | Active Ingredient | 500 |
| (b) | Hydroxypropylmethylcellulose (Methocel K4M Premium®) | 112 |
| (c) | Lactose B.P. | 53 |
| (d) | Povidone B.P.C. | 28 |
| (e) | Magnesium Stearate | <u>7</u> |
|  |  | 700 |

Drug release took place over a period of about 6-8 hours and was complete after 12 hours.

Reference Example 2: Capsule Formulations

Formulation A

A capsule formulation was prepared by admixing the ingredients of Formulation D in Example 1 above and filling into a two-part hard gelatin capsule. Formulation B (infra) was prepared in a similar manner.

Formulation B

7

|  |  |  | mg/capsule |
|---|---|---|---|
| (a) | Active ingredient | | 250 |
| (b) | Lactose B.P. | | 143 |
| (c) | Sodium Starch Glycollate | | 25 |
| (d) | Magnesium Stearate | | 2 |
| | | | 420 |

Formulation C

|  |  | mg/capsule |
|---|---|---|
| (a) | Active ingredient | 250 |
| (b) | Macrogol 4000 BP® | 350 |
| | | 600 |

Capsules were prepared by melting the Macrogol 4000 BP, dispersing the active ingredient in the melt and filling the melt into a two-part hard gelatin capsule.

Formulation D

|  | mg/capsule |
|---|---|
| Active ingredient | 250 |
| Lecithin | 100 |
| Arachis Oil | 100 |
| | 450 |

Capsules were prepared by dispersing the active ingredient in the lecithin and arachis oil and filling the dispersion into soft, elastic gelatin capsules.

Formulation E (Controlled Release Capsule)

The following controlled release capsule formulation was prepared by extruding ingredients a, b and c using an extruder, followed by spheronisation of the extrudate and drying. The dried pellets were then coated with release- controlling membrane (d) and filled into a two-piece, hard gelatin capsule.

|  | mg/capsule |
|---|---|
| (a)  Active Ingredient | 250 |
| (b)  Microcrystalline Cellulose | 125 |
| (c)  Lactose BP | 125 |
| (d)  Ethyl Cellulose | 13 |
|  | 513 |

Reference Example 3: Injectable Formulation

Formulation A.

| Active ingredient | | 0.200g |
|---|---|---|
| Hydrochloric acid solution, 0.1M | q.s. to pH | 4.0 to 7.0 |
| Sodium hydroxide solution, 0.1M | q.s. to pH | 4.0 to 7.0 |
| Sterile water | q.s. to | 10ml |

The active ingredient was dissolved in most of the water (35°-40°C) and the pH adjusted to between 4.0 and 7.0 with the hydrochloric acid or the sodium hydroxide as appropriate. The batch was then made up to volume with the water and filtered through a sterile micropore filter into a sterile 10ml amber glass vial (type 1) and sealed with sterile closures and overseals.

Formulation B

| Active ingredient | 0.125 g |
|---|---|
| Sterile, pyrogen-free, pH 7 phosphate buffer, q.s. to | 25 ml |

Reference Example 4: Intramuscular injection

| Active Ingredient | | 0.20 g |
|---|---|---|
| Benzyl Alcohol | | 0.10 g |
| Glycofurol 75 ® | | 1.45 g |
| Water for Injection | q.s. to | 3.00 ml |

The active ingredient was dissolved in the glycofurol. The benzyl alcohol was then added and dissolved, and water added to 3 ml. The mixture was then filtered through a sterile micropore filter and sealed in sterile 3 ml amber glass vials (type 1).

Reference Example 5: Ingredients

| Active ingredient | 0.2500 g |
|---|---|
| Sorbitol Solution | 1.5000 g |
| Glycerol | 2.0000 g |
| Sodium Benzoate | 0.0050 g |
| Flavour, Peach 17.42.3169 | 0.0125 ml |
| Purified Water        q.s. to | 5.0000 ml |

The active ingredient was dissolved in a mixture of the glycerol and most of the purified water. An aqueous solution of the sodium benzoate was then added to the solution, followed by addition of the sorbitol solution and finally the flavour. The volume was made up with purified water and mixed well.

Reference Example 6: Suppository

|  | mg/suppository |
|---|---|
| Active Ingredient (63$\mu$m)* | 250 |
| Hard Fat, BP (Witepsol H15 - Dynamit Nobel) | 1770 |
|  | 2020 |

* The active ingredient was used as a powder wherein at least 90% of the particles were of 63$\mu$m diameter or less.

One-fifth of the Witepsol H15 was melted in a steam-jacketed pan at 45°C maximum. The active ingredient was sifted through a 200$\mu$m sieve and added to the molten base with mixing, using a silverson fitted with a cutting head, until a smooth dispersion was achieved. Maintaining the mixture at 45°C, the remaining Witepsol H15 was added to the suspension and stirred to ensure a homogenous mix. The entire suspension was passed through a 250$\mu$m stainless steel screen and, with continous stirring, was allowed to cool to 40°C. At a temperature of 38°C to 40°C 2.02g of the mixture was filled into suitable plastic moulds. The suppositories were allowed to cool to room temperature.

Reference Example 7: Pessaries

|  | mg/pessary |
|---|---|
| Active ingredient 63$\mu$m | 250 |
| Anhydrate Dextrose | 380 |
| Potato Starch | 363 |
| Magnesium Stearate | 7 |
|  | 1000 |

The above ingredients were mixed directly and pessaries prepared by direct compression of the resulting mixture.

Reference Example 8: 3′-Azido-3′-deoxythymidine

a) 2,3′-Anhydrothymidine

Thymidine (85.4 g: 0.353 mol) was dissolved in 500 ml dry DMF and added to N-(2-chloro-1,1,2-trifluoroethyl)diethylamine (100.3 g; 0.529 mol) (prepared according to the method of D.E. Ayer, J. Med. Chem. 6, 608 (1963)). This solution was heated at 70°C for 30 minutes then poured into 950 ml ethanol (EtOH) with vigorous stirring. The product precipitated from this solution and was filtered. The EtOH supernatant was refrigerated then filtered to yield the title compound. mp. = 228 -230°C.

b) 3′-Azido-3′-deoxythymidine

2,3′-0-Anhydrothymidine (25 g: 0.1115 mol) and $NaN_3$ (29 g, 0.446 mol) was suspended in a mixture of 250 ml DMF and 38 ml water. The reaction mixture was refluxed for 5 hours at which time it was poured into 1 liter of water. The aqueous solution was extracted with EtOAc (3 x 700 ml). The EtOAc extracts were dried over $Na_2SO_4$, filtered and the EtOAc was removed in vacuo to yield a viscous oil. This oil was stirred with 200 ml water providing the title compound as a solid which was collected by filtration. mp = 116-118°C.

Example: Antiviral Activity

a) (i) Retrovirus - Induced Malignancy

3′-Azido-3′-deoxythymidine was administered to female BALB/c mice infected with $1.5X10^4$ Pfu of the RVB3 strain of Rauscher Murine Leukaemia Virus. Treatment was started 4 hours after infection at dosages of 80 mg/kg intraperitoneally every 8 hours or 0.5 or 1.0 mg/ml orally in drinking water. Such treatment was found to prevent infection of spleen cells and subsequent development of splenomegaly and also suppressed viraemia.

(ii) HTLV-I

TM-11 cells (T-cell clone susceptible to HTLV-I infection) were co-cultivated with irradiated, HTLV-I producer MJ-tumour cells as follows:
a) TM-11 cells only;
b) TM-11 cells and MJ-tumour cells
c) TM-11 cells, MJ-tumour cells and 3′-azido-3′-deoxythymidine (3$\mu$M);
d) TM-11 cells, MJ-tumour cells and 3′-azido-3′-deoxythymidine (9$\mu$M);
e) TM-11 cells, MJ-tumour cells and 3′-azido-3′-deoxythymidine (27$\mu$M).
On day 18, total DNA was extracted from each culture and digested with Bam H1 to generate a fragment of the HTLV-I genome, independent of any host flanking sequence and having a standard molecular weight of 3.3 kD. The digest was then probed with radio-labelled lambdha MT-2, a standard probe recognising the Bam H1 fragment of HTLV-I.
No hybridisation was observed for a), indicating a lack of virus in the uninfected control. A strong signal was seen for b), the untreated, infected control. A weak signal was observed with c), indicating incomplete eradication of the virus, and no hybridisation was noted in d) or e) indicating complete extermination of the virus.
Each culture was also probed with a probe for T-cell receptor $\beta$ chain, with a strong signal being generated for all cultures, showing the continued presence of TM-11 for the duration of the experiment.

(b) HTLV III

(i) In Vitro Anti-HTLV III Activity

3'-Azido-3'-deoxythymidine was tested and found to possess activity in a number of in vitro assay systems. Drug effects were measured by assaying reverse transcriptase (RT) activity in the supernates from infected, uninfected, and drug treated cells. 3'-Azido-3'-deoxythymidine effectively blocked the infection by HTLV III of the H9 and U937 human lymphoblastoid cell lines at concentrations from 2.7 to 0.0013 mcg/ml. Similarly, infection of normal PHA stimulated white blood cells and cultured peripheral blood lymphocytes was inhibited at drug concentrations as low as 0.013 mcg/ml. Drug addition and subtraction experiments in H9 cells revealed that 3'-azido-3'-deoxythymidine was most effective when present at the time of virus infection of susceptible cells, but still retained most of its antiviral activity even when added as late as 20 hours after initial HTLV III infection. Inhibition of viral replication was also evident when the drug was present in the media only during the 20 hour period of virus absorption. Effects were seen at 0.13 and 0.013 mcg/ml. 3'-Azido-3'-deoxythymidine exhibited no direct anti-RT activity against purified HTLV III virions. Similarly, the drug had little or no effect on the production and release of virions from the chronically infected H9 HTLV III cell line.

(ii) Preventing Infection by HTLV III

The ability of 3'-azido-3'-deoxythymidine to block infection of cells by HTLV III was determined as follows.

Cloned T4 positive tetanus specific T helper lymphocytes were infected with a pool of HTLV III isolates ,at challenge doses of up to 5000 virions/cell. and cell survival after infection was monitored. After 10 days in culture no viral cytopathic effects were seen in infected T cells treated with 8.8 and 1.3 mcg/ml 3'-azido-3'-deoxythymidine, while untreated, infected cells were 5-fold decreased. Cell survival was also evaluated in an HTLV-I transformed, HTLV III superinfected cell line derived from the cells above. 3'-azido-3'-deoxythymidine at concentrations of 2.7, 0.27 and 0.13 mcg/ml totally blocked cytopathic effects at 7 days. Protective effects were seen in infections induced by both cell free virions and cell associated virus. 3'-Azido-3'-deoxythymidine at 0.27 mcg/ml concentration also effectively prevented cytopathic effect induction by a less related Haitian isolate of HTLV III.

Toxicity Assay

3'-Azido-3'-deoxythymidine was administered to both mice and rats. The $LD_{50}$ value was in excess of 750 mg/kg in both species.

**Claims**

**1.** 3'-Azido-3'-deoxythymidine for use in the treatment or prophylaxis of a human retrovirus infection.

**2.** 3'-Azido-3'-deoxythymidine for use in the treatment or prophylaxis of AIDS.

**3.** 3'-Azido-3'-deoxythymidine for use in the treatment or prophylaxis of progressive generalised lymphadenopathy.

**4.** 3'-Azido-3'-deoxythymidine for use in the treatment or prophylaxis of AIDS-related complex.

**5.** 3'-Azido-3'-deoxythymidine for use in the treatment or prophylaxis of a HTLV-III infection.

**6.** 3'-Azido-3'-deoxythymidine for use in the treatment or prophylaxis of a human T-cell lymphotropic virus infection.

**7.** 3'-Azido-3'-deoxythymidine for use in the treatment or prophylaxis of a HTLV-I infection.

**8.** 3'-Azido-3'-deoxythymidine for use in the treatment or prophylaxis of a HTLV-I positive leukaemia or lymphoma.

**9.** 3'-Azido-3'-deoxythymidine for use in the treatment or prophylaxis of the HTLV-III carrier state.

**10.** 3'-Azido-3'-deoxythymidine for use in the treatment of a human subject having anti-HTLV-III antibodies.

**11.** 3'-Azido-3'-deoxythymidine as claimed in any of the preceding claims for use in conjunction with 9-[[2-hydroxy-1-(hydroxy-methyl)ethoxy]methyl]guanine, 9-(2-hydroxyethoxymethyl)guanine, 2-amino-9-(2-hydroxyethoxymethyl)purine, interferon, interleukin II or phosphonoformate.

**12.** Use of 3'-azido-3'-deoxythymidine in the manufacture of a medicament for the treatment or prophylaxis of a human retrovirus infection.

13. Use of 3'-azido-3'-deoxythymidine in the manufacture of a medicament for the treatment or prophylaxis of AIDS.

14. Use of 3'-azido-3'-deoxythymidine in the manufacture of a medicament for the treatment or prophylaxis of progressive generalised lymphadenopathy.

15. Use of 3'-azido-3'-deoxythymidine in the manufacture of a medicament for the treatment or prophylaxis of AIDS-related complex.

16. Use of 3'-azido-3'-deoxythymidine as claimed in claim 12 wherein the infection is a HTLV-III infection.

17. Use of 3'-azido-3'-deoxythymidine as claimed in claim 12 wherein the infection is a human T-cell lymphotropic virus infection.

18. Use of 3'-azido-3'-deoxythymidine as claimed in claim 12 wherein the infection is a HTLV-I infection.

19. Use of 3'-azido-3'-deoxythymidine as claimed in claim 12 wherein the infection is a HTLV-I positive leukaemia or lymphoma.

20. Use of 3'-azido-3'-deoxythymidine in the manufacture of a medicament for the treatment of the HTLV-III carrier state.

21. Use of 3'-azido-3'-deoxythymidine in the manufacture of a medicament for the treatment of a human subject having anti-HTLV-III antibodies.

**Patentansprüche**

1. 3'-Azido-3'-deoxythymidin zur Verwendung bei der Behandlung oder Prophylaxe einer Retrovirusinfektion beim Menschen.

2. 3'-Azido-3'-deoxythymidin zur Verwendung bei der Behandlung oder Prophylaxe von AIDS.

3. 3'-Azido-3'-deoxythymidin zur Verwendung bei der Behandlung oder Prophylaxe von fortgeschrittener allgemeiner Lymphadenopathie.

4. 3'-Azido-3'-deoxythymidin zur Verwendung bei der Behandlung oder Prophylaxe des mit AIDS verwandten Komplex.

5. 3'-Azido-3'-deoxythymidin zur Verwendung bei der Behandlung oder Prophylaxe einer HTLV-III-Infektion.

6. 3'-Azido-3'-deoxythymidin zur Verwendung bei der Behandlung oder Prophylaxe einer T-Zellen-lymphotropen Virusinfektion beim Menschen.

7. 3'-Azido-3'-deoxythymidin zur Verwendung bei der Behandlung oder Prophylaxe einer HTLV-I-Infektion.

8. 3'-Azido-3'-deoxythymidin zur Verwendung bei der Behandlung oder Prophylaxe einer HTLV-I-positiven Leukämie oder eines Lymphoms.

9. 3'-Azido-3'-deoxythymidin zur Verwendung bei der Behandlung oder Prophylaxe des HTLV-III-Überträgerstadium.

10. 3'-Azido-3'-deoxythymidin zur Verwendung bei der Behandlung oder Prophylaxe einer Person mit Anti-HTLV-III-Antikörper.

11. 3'-Azido-3'-deoxythymidin nach einem der vorhergehenden Ansprüche zur Verwendung bei der Konjugation mit 9-[[2-Hydroxy-1-(hydroxymethyl)ethoxy]methyl]guanin, 9-(2-Hydroxyethoxymethyl)guanin, 2-Amino-9-(2-hydroxyethoxymethyl)purin, Interferon, Interleukin II oder Phosphonoformiat.

**12.** Verwendung von 3'-Azido-3'-deoxythymidin bei der Herstellung eines Arzneimittels zur Behandlung oder Prophylaxe einer Retrovirusinfektion beim Menschen.

**13.** Verwendung von 3'-Azido-3'-deoxythymidin bei der Herstellung eines Arzneimittels zur Behandlung oder Prophylaxe von AIDS.

**14.** Verwendung von 3'-Azido-3'-deoxythymidin bei der Herstellung eines Arzneimittels zur Behandlung oder Prophylaxe einer fortschreitenden generalisierten Lymphadenopathie.

**15.** Verwendung von 3'-Azido-3'-deoxythymidin bei der Herstellung eines Arzneimittels zur Behandlung oder Prophylaxe des AIDS-verwandten Komplex.

**16.** Verwendung von 3'-Azido-3'-deoxythymidin nach Anspruch 12, worin die Infektion eine HTLV-III-Infektion ist.

**17.** Verwendung von 3'-Azido-3'-deoxythrmidin nach Anspruch 12, worin die Infektion eine T-Zellen-lymphotrope Virusinfektion des Menschen ist.

**18.** Verwendung von 3'-Azido-3'-deoxythymidin nach Anspruch 12, worin die Infektion eine HTLV-I-Infektion ist.

**19.** Verwendung von 3'-Azido-3'-deoxythymidin nach Anspruch 12, worin die Infektion eine HTLV-I-positive Leukämie oder ein Lymphom ist.

**20.** Verwendung von 3'-Azido-3'-deoxythymidin bei der Herstellung eines Arzneimittels zur Behandlung des HTLV-III-Überträgerstadiums.

**21.** Verwendung von 3'-Azido-3'-deoxythymidin bei der Herstellung eines Arzneimittels zur Behandlung eines Menschen mit Anti-HTLV-III-Antikörpern.

**Revendications**

**1.** La 3'-azido-3'-désoxythymidine pour l'utilisation dans le traitement ou la prophylaxie d'une infection rétrovirale humaine.

**2.** La 3'-azido-3'-désoxythymidine pour l'utilisation dans le traitement ou la prophylaxie du SIDA.

**3.** La 3'-azido-3'-désoxythymidine pour l'utilisation dans le traitement ou la prophylaxie de la lymphodénopathie généralisée progressive.

**4.** La 3'-azido-3'-désoxythymidine pour l'utilisation dans le traitement ou la prophylaxie des manifestations accompagnant le SIDA.

**5.** La 3'-azido-3'-désoxythymidine pour l'utilisation dans le traitement ou la prophylaxie d'une infection à HTLV-III.

**6.** La 3'-azido-3'-désoxythymidine pour l'utilisation dans le traitement ou la prophylaxie d'une infection par le virus lymphotrope des cellules T humaines.

**7.** La 3'-azido-3'-désoxythymidine pour l'utilisation dans le traitement ou la prophylaxie d'une infection à HTLV-I.

**8.** La 3'-azido-3'-désoxythymidine pour l'utilisation dans le traitement ou la prophylaxie d'une leucémie ou d'un lymphome HTLV-I-positif.

**9.** La 3'-azido-3'-désoxythymidine pour l'utilisation dans le traitement ou la prophylaxie de l'état de porteur de HTLV-III.

**10.** La 3'-azido-3'-désoxythymidine pour l'utilisation dans le traitement d'un sujet humain ayant des anticorps anti-HTLV-III.

**11.** La 3'-azido-3'-désoxythymidine suivant l'une quelconque des revendications précédentes pour l'utilisation en conjonction avec la 9-[[2-hydroxy-1-(hydroxyméthyl)éthoxy]méthyl]guanine, la 9-(2-hydroxyéthoxyméthyl)guanine, la 2-amino-9-(2-hydroxyéthoxyméthyl)purine, un interféron, l'interleukine II ou le phosphonoformate.

**12.** Utilisation de la 3'-azido-3'-désoxythymidine dans la fabrication d'un médicament pour le traitement ou la prophylaxie d'une infection rétrovirale humaine.

**13.** Utilisation de la 3'-azido-3'-désoxythymidine dans la fabrication d'un médicament pour le traitement ou la prophylaxie du SIDA.

**14.** Utilisation de la 3'-azido-3'-désoxythymidine dans la fabrication d'un médicament pour le traitement ou la prophylaxie du lymphodénopathie généralisée progressive.

**15.** Utilisation de la 3'-azido-3'-désoxythymidine dans la fabrication d'un médicament pour le traitement ou la prophylaxie des manifestations accompagnant le SIDA.

**16.** Utilisation de la 3'-azido-3'-désoxythymidine selon la revendication 12, dans laquelle l'infection est une infection à HTLV--III.

**17.** Utilisation de la 3'-azido-3'-désoxythymidine selon la revendication 12, dans laquelle l'infection est une infection par le virus lymphotrope des cellules T humaines.

**18.** Utilisation de la 3'-azido-3'-désoxythymidine selon la revendication 12 dans laquelle l'infection est une infection à HTLV-I.

**19.** Utilisation de la 3'-azido-3'-désoxythymidine selon la revendication 12, dans laquelle l'infection est une leucémie ou un lymphome HTLV-I-positif.

**20.** Utilisation de la 3'-azido-3'-désoxythymidine dans la fabrication d'un médicament pour le traitement de l'état de porteur de HTLV-III.

**21.** Utilisation de la 3'-azido-3'-désoxythymidine dans la fabrication d'un médicament pour le traitement d'un sujet humain ayant des anticorps anti-HTLV-III.